# EUROPEAN PATENT SPECIFICATION

(11) **EP 2 810 657 B1**
(45) Date of publication and mention of the grant of the patent: **21.09.2016**
(21) Application number: 13744346.1
(22) Date of filing: 30.01.2013
(51) Int. Cl.: A61K 47/16, A61K 9/10, A61K 47/10, A61K 47/32, A61P 27/02, A61K 47/22, A61K 9/00, A61K 9/127, A61K 47/24, A61K 31/513, A61K 31/573

(54) **NON-AQUEOUS LIQUID COMPOSITION**
NICHTWÄSSRIGE FLÜSSIGKEITSZUSAMMENSETZUNG
COMPOSITION LIQUIDE NON AQUEUSE

(30) Priority: 31.01.2012 JP 2012018062
(43) Date of publication of application: 10.12.2014
(73) Proprietor: Santen Pharmaceutical Co., Ltd, Osaka-shi, Osaka 533-8651 (JP)
(72) Inventor: YAMADA, Kazuhito, Ikoma-shi Nara 630-0101 (JP); URTTI, Arto, FI-70420 Kuopio (FI); BURMESTER, Mechthild, FI-00760 Helsinki (FI)
(74) Representative: Müller-Boré & Partner Patentanwälte PartG mbB
(86) International application number: PCT/JP2013/051951
(87) International publication number: WO 2013/115201

(56) References cited:
- JP-A- 2001 524 958
- JP-A- 2007 527 869
- JP-A- 2008 501 676
- JP-A- 2008 526 932
- JP-A- 2008 526 933
- US-A1- 2005 112 188
- US-A1- 2008 274 176

## Description

### Non-Aqueous Liquid Composition

The present invention relates to a drug-dissolved non-aqueous liquid composition containing a drug, dioleylphosphatidylcholine, tocopherol and an organic solvent, and an injectable solution and an ophthalmic preparation containing the non-aqueous liquid composition.

Dioleylphosphatidylcholine (hereinafter, "DOPC") is an amphiphile having such a structure that two oleic acids, glycerin, phosphoric acid and choline are combined. As an amphiphile, besides DOPC, for example, dioleylphosphatidylglycerin (hereinafter, "DOPG"), dioleylphosphatidylethanolamine (hereinafter, "DOPE"), phosphatidylcholine (hereinafter, "PC"), soybean lecithin (hereinafter, "SPC"), glycerin monooleate (hereinafter, "GMO") and so on are known.

On the other hand, for administration of a drug to a living body, it is demanded to develop depot preparations having the action of staying in the vicinity of the administration site (such as a vitreous body) for a long term, and continuously releasing the drug. For example, WO 2006/131730 (Patent Document 1) discloses a depot preparation containing SPC and dioleylglycerol (hereinafter, "GDO"), and WO 2005/117830 (Patent Document 2) discloses a depot preparation containing PC and GDO, and a depot preparation containing PC and tocopherol (hereinafter, "VE"). However, the depot preparations disclosed in Patent Documents 1 and 2 are not satisfactory in terms of sustained releasability of a drug in a living body on the assumption that they are administered to a living body such as in a vitreous body, and also have a risk of swelling in the living body.
PTD 1: WO 2006/131730
PTD 2: WO 2005/117830
Further, US 2008/274176A1 describes pharmaceutical lipid compositions, and US 2005/0112188 A1 describes composition and dosage form comprising an amphiphilic molecule as a suspension vehicle.

The present invention was made for solving the above problems, and it is an object of the present invention to search for a non-aqueous preparation that is in a liquid state before administration, and changes into a depot (liquid crystal state) and has a drug sustained-release action in a living body after administration, by utilizing the characteristics of DOPC which is an amphiphile.

Inventors of the present application found that a non-aqueous liquid composition containing a drug, DOPC, VE and an organic solvent, wherein the blend concentration ratio between DOPC and VE falls within a specific range is a viscous liquid, but it forms a non-lamellar liquid crystal upon contact with a phosphate buffer or a vitreous fluid and continuously releases the drug for a long term in the living body; and accomplished the present invention. More specifically, the present invention is as follows.
(1) A drug-dissolved non-aqueous liquid composition containing a drug, DOPC, VE and an organic solvent selected from ethanol, propylene glycol, glycerin, polyethylene glycol, benzyl alcohol, dimethylacetamide and dimethylsulfoxide, wherein
   1) the blend concentration ratio between the DOPC and the VE falls within the range of 75/25 to 25/75,
   2) the blend concentration of the DOPC falls within the range of 15 to 85% (w/w),
   3) the blend concentration of the VE falls within the range of 15 to 85% (w/w), and
   4) the phase of the non-aqueous liquid composition changes into a non-lamellar liquid crystal upon contact with water, a phosphate buffer, a body fluid, a lacrimal fluid or a vitreous fluid.
(2) The drug-dissolved non-aqueous liquid composition according to (1), wherein
   1) the blend concentration ratio between the DOPC and the VE falls within the range of 70/30 to 30/70,
   2) the blend concentration of the DOPC falls within the range of 20 to 80% (w/w), and
   3) the blend concentration of the VE falls within the range of 20 to 80% (w/w).
(3) The non-aqueous liquid composition according to (1) or (2), wherein the organic solvent is ethanol, benzyl alcohol, polyethylene glycol or dimethylacetamide.
(4) An injectable solution containing the non-aqueous liquid composition according to any one of (1) to (3).
(5) An ophthalmic preparation containing the non-aqueous liquid composition according to any one of (1) to (3).

As evidenced by the later-described phase behavior test, and in vitro and in vivo drug release characteristics test, the non-aqueous liquid composition of the present invention undergoes phase transition into a non-lamellar liquid crystal to form a solid depot upon contact with a phosphate buffer, a vitreous fluid or the like, and as a result, it sustainedly releases a drug stably for a long term. Thus, the non-aqueous liquid composition of the present invention is expected to exert an excellent drug sustained release effect by undergoing phase transition into a non-lamellar liquid crystal after administration although it is a liquid which is easy to handle before administration to a living body. Further, as indicated by the results of a swelling property test using various amphiphiles as will be described later, there is no risk of causing side effects such as a visual field disorder or reduction in visual acuity owing to swelling because the non-aqueous liquid composition of the present invention containing DOPC as an amphiphile did not swell in water for 4 months or longer when it was administered to, for example, a vitreous body.

The drug in the non-aqueous liquid composition of the present invention is not particularly limited, and is desirably a drug that is soluble in a non-aqueous liquid containing DOPC, VE and an organic solvent. Examples of a preferred drug include steroids such as hydrocortisone, triamcinolone, fluocinolone and dexamethasone, prostaglandins such as isopropyl unoprostone, immunosuppressive agents such as cyclosporine and rapamycin, nonsteroidal anti-inflammatory drugs such as indomethacin and bromfenac, angiogenesis inhibitors such as pazopanib, SU5416, valatinib, ranibizumab and bevacizumab, VEGF inhibitors as described in Japanese Patent Laying-Open No. 2006-96739, Japanese Patent Laying-Open No. 2011-37844, Japanese Patent Laying-Open No. 2005-232149, Japanese Patent Laying-Open No. 2006-273851, Japanese Patent Laying-Open No. 2006-306861, Japanese Patent Laying-Open No. 2008-266294 and so on, compounds having glucocorticoid receptor binding activity as described in Japanese Patent Laying-Open No. 2007-230993, Japanese Patent Laying-Open No. 2008-074829, Japanese Patent Laying-Open No. 2008-143889, Japanese Patent Laying-Open No. 2008-143890, Japanese Patent Laying-Open No. 2008-143891, Japanese Patent Laying-Open No. 2009-007344, Japanese Patent Laying-Open No. 2009-084274 and so on, selective glucocorticoid receptor agonists such as RU24858, anticancer agents such as fluorouracil, janus kinase inhibitors such as tofacitinib, and protein kinase inhibitors such as ruboxistaurin mesylate.

The blend concentration of the drug in the non-aqueous liquid composition of the present invention is not particularly limited because it differs depending on the kind of the drug, and it is preferably within the range of 0.1 to 60% (w/w), more preferably within the range of 0.1 to 10% (w/w), and particularly preferably within the range of 0.2 to 8% (w/w).

DOPC in the non-aqueous liquid composition of the present invention is an amphiphile, and its blend concentration is within the range of 15 to 85% (w/w), and preferably within the range of 20 to 80% (w/w).

VE in the non-aqueous liquid composition of the present invention means α-tocopherol (vitamin E), β-tocophérol, or γ-tocopherol, and may be a tocopherol derivative such as tocopherol acetate, tocopherol nicotinate, or tocopherol succinate. The blend concentration of VE in the non-aqueous liquid composition of the present invention is within the range of 15 to 85% (w/w), and preferably within the range of 20 to 80% (w/w).

The blend concentration ratio between DOPC and VE in the non-aqueous liquid composition of the present invention is within the range of 75/25 to 25/75, preferably within the range of 70/30 to 30/70, and more preferably within the range of 70/30 to 35/65. The result of the later-described phase behavior test revealed that when the blend concentration ratio between DOPC and VE falls within such a range, the non-aqueous liquid composition, which is originally a viscous liquid, undergoes phase transition into a non-lamellar liquid crystal upon contact with the phosphate buffer or the vitreous fluid to form a hard depot. Here, the term "non-lamellar liquid crystal" means a liquid crystal in the form of not having a lamellar structure (laminar structure) in the liquid crystal state that is between the liquid and the solid, and examples of the non-lamellar liquid crystal include a reversed hexagonal liquid crystal (H2) and a reversed cubic liquid crystal (Q2). Whether or not the phase has transitioned to a non-lamellar liquid crystal can be confirmed by observation under a polarizing microscope: an anisotropic striped pattern or geometric pattern is observed due to birefringence in the case of the reversed hexagonal liquid crystal, and a dark field is observed due to absence of birefringence in the case of the reversed cubic liquid crystal. The non-aqueous liquid composition of the present invention exhibited the stable drug sustained releasability for 70 days or longer in the later-described in vitro drug release characteristics test and for 12 weeks (84 days) in the later-described in vivo drug release characteristics test, revealing that it is useful as a depot preparation. Thus, the stable drug sustained releasability of the non-aqueous liquid composition of the present invention is an effect that is never achieved without making the blend concentration ratio between DOPC and VE fall within the above range.

The organic solvent used in the non-aqueous liquid composition of the present invention is a pharmaceutically acceptable organic solvent selected from ethanol, propylene glycol, glycerin, polyethylene glycols such as PEG400, benzyl alcohol, dimethylacetamide (DMA) and dimethylsulfoxide (DMSO). Among these, ethanol, benzyl alcohol, polyethylene glycol or dimethylacetamide is preferred.

Although not particularly limited, the blend concentration of the organic solvent in the non-aqueous liquid composition of the present invention is preferably within the range of 1 to 50% (w/w), and more preferably within the range of 3 to 30% (w/w).

The non-aqueous liquid composition of the present invention is preferably administered parenterally, and as the dosage forms, for example, a liquid medicine, an injectable solution and so on are recited, and these may be formulated by using generally used techniques. For example, liquid medicines, injectable solutions and so on may be prepared by using surfactants such as polyoxyethylene sorbitan monooleate, polyoxyl 40 stearate and polyoxyethylene hardened castor oil, stabilizing agents such as sodium edetate, and antiseptics such as benzalkonium chloride and paraben as necessary. The non-aqueous liquid composition of the present invention can be utilized as an injectable solution or an ophthalmic preparation containing the same.

Hereinafter, results of various tests and preparation examples will be shown, however, it is to be noted that they are given for better understanding of the present invention and not for limiting the scope of the present invention.

### [1] Phase behavior test

To compositions having various blend concentration ratios between dioleylphosphatidylcholine (DOPC) and tocopherol (VE), a phosphate buffer was added and the phase behavior was examined.

### (Experimental procedure)

DOPC was dissolved in methanol to have a concentration of 500 mg/mL, and VE was dissolved in methanol to have a concentration of 500 mg/mL. The prepared DOPC solution and VE solution were mixed in the proportions as shown in Table 1 shown below, and methanol was removed under nitrogen gas flow, and the mixture was stored for 24 hours or longer under reduced pressure. After the storage, a phosphate buffer (pH 7.4) was added and mixed with the resultant composition, and the appearance and the phase behavior of the composition were confirmed by using a polarizing microscope (LEICA DMLB, available from LICA). The phosphate buffer was added in increments of 5% (w/w) until the concentration of the phosphate buffer in the DOPC/VE became 45% (w/w).

### (Results)

The appearance and the phase behavior after addition of the phosphate buffer are shown in Table 1.

**[Table 1]**

| Composition | DOPC (w/w) | VE (w/w) | DOPC/VE | Appearance | Phase behavior after addition of phosphate buffer |
|---|---|---|---|---|---|
| 1 | 100 | 0 | 100/0 | Viscous liquid | Did not form liquid crystal |
| 2 | 90 | 10 | 90/10 | Viscous liquid | Did not form liquid crystal |
| 3 | 80 | 20 | 80/20 | Viscous liquid | Did not form liquid crystal |
| 4 | 70 | 30 | 70/30 | Nonfluid mass | Q2^{*1} |
| 5 | 60 | 40 | 60/40 | Nonfluid mass | Q2 |
| 6 | 50 | 50 | 50/50 | Nonfluid mass | H2^{*2} |
| 7 | 40 | 60 | 40/60 | Nonfluid mass | H2 |
| 8 | 30 | 70 | 30/70 | Nonfluid mass | H2 |
| 9 | 20 | 80 | 20/80 | Viscous liquid | Did not form liquid crystal |
| 10 | 10 | 90 | 10/90 | Viscous liquid | Did not form liquid crystal |
| 11 | 0 | 100 | 0/100 | Viscous liquid | Did not form liquid crystal |

| | | | | | |
|---|---|---|---|---|---|
| *1: Q2 indicates reversed cubic liquid crystal. *2: H2 indicates reversed hexagonal liquid crystal. | | | | | |

### (Discussion)

From Table 1, it is revealed that Compositions 4 to 8 included in the present invention will form depots when administered to a living body because they form a non-lamellar liquid crystal Q2 or H2. On the other hand, it is expected that Compositions 1 to 3 and 9 to 11 not included in the present invention will not form a depot when they are administered to a living body because they do not form a liquid crystal.

### [2] Preparation Examples 1 to 6 and Comparative Examples 1 to 4

### (1) Preparation Example 1

To 20 mg of hydrocortisone (HC), about 980 mg of ethanol (EtOH) was added and stirred until it was dissolved under warming to 60°C (2% HC solution). This 50 mg of 2% HC solution was added to 225 mg of DOPC, and dissolved under stirring under warming to 70°C. After dissolution, 225 mg of VE was added, and stirred until it was dissolved under warming to 70°C again to prepare a 0.2% HC-containing DOPC preparation.

### (2) Preparation Example 2

A 0.2% TA-containing DOPC preparation was prepared by a procedure similar to that of Preparation Example 1 except that 20 mg of triamcinolone acetonide (TA) was used in place of HC.

### (3) Preparation Example 3

A 0.2% FA-containing DOPC preparation was prepared by a procedure similar to that of Preparation Example 1 except that 20 mg of fluorocinolone acetonide (FA) was used in place of HC.

### (4) Preparation Example 4

To 50 mg of FA, about 200 mg of benzyl alcohol (BzOH) was added, and stirred until it was dissolved under warming to 60°C (20% FA solution). This 100 mg of 20% FA solution was added to 200 mg of DOPC, and dissolved under stirring under warming to 70°C. After dissolution, 200 mg of VE was added, and stirred until it was dissolved under warming to 70°C again to prepare a 4% FA-containing DOPC preparation.

### (5) Preparation Example 5

To 294 mg of polyethylene glycol (PEG) 400, 6 mg of fluorouracil (5-FU) was added, and stirred until it was dissolved under warming to 70°C (2% 5-FU solution). This 50 mg of 2% 5-FU solution was added to 225 mg of DOPC, and dissolved under stirring under warming to 70°C. After dissolution, 225 mg of VE was added, and stirred until it was dissolved under warming to 70°C again to prepare a 0.2% 5-FU-containing DOPC preparation.

### (6) Preparation Example 6

To 294 mg of PEG400, 6 mg of 5-FU was added and stirred until it was dissolved under warming to 70°C (2% 5-FU solution). This 50 mg of 2% 5-FU solution was added to 180 mg of DOPC, and dissolved under stirring under warming to 70°C. After dissolution, 270 mg of tocopherol was added, and stirred until it was dissolved under warming to 70°C again to prepare a 0.2% 5-FU-containing DOPC preparation.

### (7) Comparative Example 1

To 20 mg of TA, about 980 mg of EtOH was added, and stirred until it was dissolved under warming to 60°C (2% TA solution). This 50 mg of 2% TA solution was added to 225 mg of soybean lecithin (SPC), and dissolved under stirring under warming to 70°C. After dissolution, 225 mg of VE was added, and stirred until it was dissolved under warming to 70°C again to prepare a 0.2% TA-containing SPC preparation.

### (8) Comparative Example 2

To 90 mg of SPC, 50 mg of the 2% TA solution prepared in Comparative Example 1 was added, and dissolved under stirring under warming to 70°C. After dissolution, 90 mg of dioleyl glycerol (GDO) was added, and stirred until it was dissolved under warming to 70°C again to prepare a 0.2% TA-containing SPC preparation.

### (9) Comparative Example 3

Glycerin monooleate (GMO) was dissolved under warming at 40°C. After dissolution, 50 mg of the 2% TA solution prepared in Comparative Example 1 was added to 225 mg of GMO, and dissolved under stirring under warming to 40°C. After dissolution, 225 mg of VE was added, and stirred until it was dissolved under warming to 40°C again to prepare a 0.2% TA-containing GMO preparation.

### (10) Comparative Example 4

GMO was dissolved under warming at 40°C. After dissolution, 50 mg of the 2% TA solution prepared in Comparative Example 1 was added to 450 mg of GMO, and dissolved under stirring under warming to about 40°C to prepare a 0.2% TA-containing GMO preparation.

### [3] Phase behavior test of each preparation obtained in Preparation Examples 1 to 6 and Comparative Examples 1 to 4.

### (Experimental procedure)

Each 20 mg of the respective solution of Preparation Examples 1 to 6 and Comparative Examples 1 to 4 prepared in the above item [2] was put into 2 mL of a 0.1 M phosphate buffer (pH 7.4) and incubated at 37°C. All preparations formed hard depots after being put into the phosphate buffer. After one day, the phase behaviors of the generated depots were evaluated by using a polarizing microscope.

### (Results of Preparation Examples 1 to 6)

The phase behaviors of Preparation Examples 1 to 6 are shown in Table 2. The numerical values of ingredients shown in Table 2 are represented by % (w/w).

**[Table 2]**

| | DOPC | VE | Organic solvent | Drug | Phase after put into phosphate buffer |
|---|---|---|---|---|---|
| Preparation Example 1 | 45 | 45 | EtOH 9.8 | HC 0.2 | H2 |
| Preparation Example 2 | 45 | 45 | EtOH 9.8 | TA 0.2 | H2 |
| Preparation Example 3 | 45 | 45 | EtOH 9.8 | FA 0.2 | H2 |
| Preparation Example 4 | 40 | 40 | BzOH 16.0 | FA 4.0 | H2 |
| Preparation Example 5 | 45 | 45 | PEG400 9.8 | 5-FU 0.2 | H2 |
| Preparation Example 6 | 36 | 54 | PEG400 9.8 | 5-FU 0.2 | H2/Q2 |

### (Results of Comparative Examples 1 to 4)

The phase behaviors of Comparative Examples 1 to 4 are shown in Table 3. Ingredients in Table 3 are represented by % (w/w).

**[Table 3]**

| | SPC | GMO | VE | GDO | EtOH | TA | Phase after put into phosphate buffer |
|---|---|---|---|---|---|---|---|
| Comparative Example 1 | 45 | | 45 | | 9.8 | 0.2 | H2 |
| Comparative Example 2 | 45 | | | 45 | 9.8 | 0.2 | Q2 |
| Comparative Example 3 | | 45 | 45 | | 9.8 | 0.2 | Q2 |
| Comparative Example 4 | | 90 | | | 9.8 | 0.2 | Q2 |

### [4] Preparation Examples 7 to 34 and phase behavior tests for the preparations

An experimental procedure similar to that in Preparation Examples 1 to 6 was conducted to prepare preparations having compositions as shown in Table 4 below (Preparation Examples 7 to 34), and the phase behaviors thereof were evaluated. The results are also shown in Table 4. The numerical values of ingredients in Table 4 are represented by % (w/w).

**[Table 4]**

| | DOPC | VE | Organic solvent | Drug | Phase after put into phosphate buffer |
|---|---|---|---|---|---|
| Preparation Example 7 | 54 | 36 | PG ^{*3}9.8 | DSP ^{*4}0.2 | H2 |
| Preparation Example 8 | 54 | 36 | PG 10.0 | | H2 |
| Preparation Example 9 | 54 | 36 | EtOH 9.8 | TA 0.2 | H2 |
| Preparation Example 10 | 54 | 36 | EtOH 10.0 | | H2 |
| Preparation Example 11 | 45 | 45 | EtOH 9.8 | TA 0.2 | H2 |
| Preparation Example 12 | 45 | 45 | EtOH 10.0 | | H2 |
| Preparation Example 13 | 53 | 27 | EtOH 19.8 | TA 0.2 | H2 |
| Preparation Example 14 | 53 | 27 | EtOH 20.0 | | H2 |
| Preparation Example 15 | 48 | 32 | EtOH 19.8 | TA 0.2 | H2 |
| Preparation Example 16 | 48 | 32 | EtOH 20.0 | | H2 |
| Preparation Example 17 | 40 | 40 | EtOH 19.8 | TA 0.2 | H2 |
| Preparation Example 18 | 40 | 40 | EtOH 20.0 | | Q2 |
| Preparation Example 19 | 54 | 36 | BzOH 9.8 | TA 0.2 | H2 |
| Preparation Example 20 | 54 | 36 | BzOH 10.0 | | H2 |
| Preparation Example 21 | 48 | 32 | BzOH 19.8 | TA 0.2 | H2 |
| Preparation Example 22 | 48 | 32 | BzOH 20.0 | | H2 |
| Preparation Example 23 | 54 | 36 | EtOH 9.8 | TA 0.2 | H2 |
| Preparation Example 24 | 48 | 32 | EtOH 10.0 | | H2 |
| Preparation Example 25 | 54 | 36 | PG 9.8 | TA 0.2 | H2 |
| Preparation Example 26 | 48 | 32 | PG 19.8 | TA 0.2 | H2 |
| Preparation Example 27 | 48 | 32 | PG 20.0 | | H2 |
| Preparation Example 28 | 48 | 32 | PG 19.8 | TA 0.2 | H2/Q2 |
| Preparation Example 29 | 40 | 40 | PG 20.0 | | H2/Q2 |
| Preparation Example 30 | 54 | 36 | BzOH 9.8 | TA 0.2 | H2 |
| Preparation Example 31 | 48 | 32 | BzOH 19.8 | TA 0.2 | H2 |
| Preparation Example 32 | 40 | 40 | BzOH 19.8 | TA 0.2 | H2 |
| Preparation Example 33 | 40 | 40 | BzOH 20.0 | | Q2 |
| Preparation Example 34 | 48 | 32 | BzOH 16.0 | TA 4.0 | H2 |

| | | | | | |
|---|---|---|---|---|---|
| *3: PG represents propylene glycol. *4: DSP represents dexamethasone phosphate. | | | | | |

### (Discussion)

Table 4 reveals that all of Preparation Examples 7 to 34 form a non-lamellar liquid crystal Q2 or H2.

### [5] In vitro drug release characteristics test for each preparation obtained in Preparation Examples 1 to 6 and Comparative Examples 1 to 4

### (Experimental procedure)

Each 20 mg of the respective solution prepared in Preparation Examples 1 to 6 and Comparative Examples 1 to 4 was put into 2 mL of a 0.1 M phosphate buffer (pH 7.4) and incubated at 37°C. All of the preparations formed hard depots after being put into the phosphate buffer. The phosphate buffer was collected over time, and the drug concentration in the phosphate buffer was measured by using UPLC.

### (Results)

In vitro drug release characteristics of each preparation are shown in Table 5. Each cumulative drug release rate (%) in Table 5 is a mean value of three cases, and "ND" indicates that the result is below the detection limit.

**[Table 5]**

| | Drug | Cumulative drug release rate (%) | | | | |
|---|---|---|---|---|---|---|
| | | After 1 day | After 7 days | After 28 days | After 56 days | After 70 days |
| Preparation Example 1 | HC | 4.6 | 14.1 | 27.8 | 39.6 | 43.2 |
| Preparation Example 2 | TA | 6.1 | 19.1 | 36.4 | 48.7 | 53.1 |
| Preparation Example 3 | FA | 4.5 | 12.8 | 26.0 | 35.6 | 39.3 |
| Preparation Example 4 | FA | 10.1 | 19.0 | 32.3 | 42.7 | 46.4 |
| Preparation Example 5 | 5-FU | 10.8 | 39.2 | 63.7 | 72.9 | 74.2 |
| Preparation Example 6 | 5-FU | 28.0 | 54.5 | 73.0 | 73.9 | 73.9 |
| Comparative Example 1 | TA | 8.6 | 24.1 | 48.7 | 62.7 | ND |
| Comparative Example 2 | TA | 14.3 | 37.2 | 62.3 | 68.0 | ND |
| Comparative Example 3 | TA | 34.0 | 60.3 | 82.1 | ND | ND |
| Comparative Example 4 | TA | 41.2 | 64.1 | 84.1 | ND | ND |

### (Discussion)

As can be seen from Table 5, since DOPC preparations (Preparation Examples 1 to 6) continuously release the drug for 70 days or longer for any of the four kinds of drugs, they are expected to exert a better drug sustained release effect than the SPC preparations (Comparative Examples 1 and 2) and the GMO preparations (Comparative Examples 3 and 4).

### [6] In vivo drug release characteristics test

### (Experimental procedure)

To 80 mg of triamcinolone acetonide (TA), 720 mg of dimethylacetamide (DMA) was added and stirred until it was dissolved (10% TA solution). To this 10% TA solution, 1600 mg of DOPC and 1600 mg of tocopherol were added, and stirred until they were dissolved under warming to 65°C to prepare a 10% TA-containing DOPC preparation (Preparation Example 35), and the phase behavior after it was put into a phosphate buffer was evaluated. The phase behavior of Preparation Example 35 is shown in Table 6. The numerical values of ingredients in Table 6 are represented by % (w/w).

**[Table 6]**

| | DOPC | VE | Organic solvent | Drug | Phase after put into phosphate buffer |
|---|---|---|---|---|---|
| Preparation Example 35 | 40 | 40 | DMA 18 | TA 2 | H2 |

Next, 50 µL of Preparation Example 35 was administered to the vitreous body of Japanese white rabbit (male) with the use of a 26-gauge needle (N = 4). Aqueous humor, vitreous body, and choroid were sampled from the rabbits over time, and the drug concentration was measured by using LC-MS/MS. The measurement results of the drug concentration of Preparation Example 35 are shown in Table 7.

**[Table 7]**

| Time after injection (day) | Concentration in aqueous humor (ng/mL) | Concentration in vitreous body (µg/g) | Concentration in choroid (µg/g) |
|---|---|---|---|
| 4 | 36.9 | 1.54 | 9.48 |
| 7 | 19.4 | 0.88 | 2.75 |
| 28 | 2.17 | 0.19 | 0.55 |
| 56 | 0.98 | 0.07 | 0.18 |
| 84 | 0.70 | 0.06 | 0.08 |

### (Discussion)

Since the present preparation (Preparation Example 35) keeps above a certain triamcinolone acetonide concentration for 12 weeks (84 days) after injection into the vitreous body, it is expected to maintain the drug sustained release effect for a long term.

### [7] Phase behaviors and swelling properties of various compositions obtained from various amphiphiles and VE

### (Experimental procedure)

As shown in Table 8 below, each 90 mg of DOPC, DOPG, DOPE or SPC as an amphiphile was collected, and 10 mg of EtOH and 90 mg of VE were added. After warming at about 70°C, these were stirred and dissolved. After dissolution, 0.1 mL of each composition was put into 2 mL of water, and incubated at 37°C. All the compositions formed hard depots after being put into water. After one day, the phase behaviors of the formed depots were evaluated by using a polarizing microscope. Also, whether the depots swell in the water was evaluated. As a result, while all the compositions formed hard depots formed of a non-lamellar liquid crystal, swelling in water was not observed for the compositions containing DOPC, and swelling in the water was observed for the compositions containing DOPG, DOPE or SPC. The numerical values of ingredients in Table 8 are represented by % (w/w).

**[Table 8]**

| Composition | Amphiphile | VE | EtOH | Phase behavior (After 1 day) | Swelling | |
|---|---|---|---|---|---|---|
| | | | | | (After 1 day) | (After 4 months) |
| 12 | DOPC 45 | 45 | 10 | H2 | Not observed | Not observed |
| 13 | DOPG 45 | 45 | 10 | Q2 | Not observed | Observed |
| 14 | DOPE 45 | 45 | 10 | Q2 | Not observed | Observed |
| 15 | SPC 45 | 45 | 10 | Q2 | Not observed | Observed |

### (Discussion)

DOPC preparations are suited for administration to a vitreous body or a body fluid because a preparation (composition) that swells in water can cause a visual field disorder or reduction in visual acuity of a patient when it is administered to the vitreous body.

## Claims

1. A drug-dissolved non-aqueous liquid composition comprising a drug, dioleylphosphatidylcholine, tocopherol and an organic solvent selected from ethanol, propylene glycol, glycerin, polyethylene glycol, benzyl alcohol, dimethylacetamide and dimethylsulfoxide, wherein
1) the blend concentration ratio between said dioleylphosphatidylcholine and said tocopherol falls within the range of 75/25 to 25/75,
2) the blend concentration of said dioleylphosphatidylcholine falls within the range of 15 to 85% (w/w),
3) the blend concentration of said tocopherol falls within the range of 15 to 85% (w/w), and
4) the phase of the non-aqueous liquid composition changes into a non-lamellar liquid crystal upon contact with water, a phosphate buffer, a body fluid, a lacrimal fluid or a vitreous fluid.

2. The drug-dissolved non-aqueous liquid composition according to claim 1, wherein
1) the blend concentration ratio between said dioleylphosphatidylcholine and said tocopherol falls within the range of 70/30 to 30/70,
2) the blend concentration of said dioleylphosphatidylcholine falls within the range of 20 to 80% (w/w), and
3) the blend concentration of said tocopherol falls within the range of 20 to 80% (w/w).

3. The non-aqueous liquid composition according to claim 1 or 2, wherein said organic solvent is ethanol, benzyl alcohol, polyethylene glycol or dimethylacetamide.

4. An injectable solution comprising the non-aqueous liquid composition according to any one of claims 1 to 3.

5. An ophthalmic preparation comprising the non-aqueous liquid composition according to any one of claims 1 to 3.

## Patentansprüche

1. Nicht-wässrige flüssige Zusammensetzung mit gelöstem Wirkstoff, umfassend einen Wirkstoff, Dioleylphosphatidylcholin, Tocopherol und ein organisches Lösungsmittel, ausgewählt aus Ethanol, Propylenglycol, Glycerin, Polyethylenglycol, Benzylalkohol, Dimethylacetamid und Dimethylsulfoxid, wobei
1) das Mischungskonzentrationsverhältnis zwischen dem Dioleylphosphatidylcholin und dem Tocopherol in den Bereich von 75/25 bis 25/75 fällt,
2) die Mischungskonzentration des Dioleylphosphatidylcholins in den Bereich von 15 bis 85% (w/w) fällt,
3) die Mischungskonzentration des Tocopherols in den Bereich von 15 bis 85% (w/w) fällt, und
4) die Phase der nicht-wässrigen flüssigen Zusammensetzung bei Kontakt mit Wasser, einem Phosphatpuffer, einer Körperflüssigkeit, einer Tränenflüssigkeit oder einer Glaskörperflüssigkeit zu einem nicht-lamellaren Flüssigkristall wechselt.

2. Nicht-wässrige flüssige Zusammensetzung mit gelöstem Wirkstoff nach Anspruch 1, wobei
1) das Mischungskonzentrationsverhältnis zwischen dem Dioleylphosphatidylcholin und dem Tocopherol in den Bereich von 70/30 bis 30/70 fällt,
2) die Mischungskonzentration des Dioleylphosphatidylcholins in den Bereich von 20 bis 80% (w/w) fällt, und
3) die Mischungskonzentration des Tocopherols in den Bereich von 20 bis 80% (w/w) fällt.

3. Nicht-wässrige flüssige Zusammensetzung nach Anspruch 1 oder 2, wobei das organische Lösungsmittel Ethanol, Benzylalkohol, Polyethylenglycol oder Dimethylacetamit ist.

4. Injizierbare Lösung, umfassend die nicht-wässrige flüssige Zusammensetzung nach einem der Ansprüche 1 bis 3.

5. Ophthalmische Zubereitung, umfassend die nicht-wässrige flüssige Zusammensetzung nach einem der Ansprüche 1 bis 3.

## Revendications

1. Composition liquide non aqueuse dissoute dans un médicament, comprenant un médicament, de la dioleylphosphatidylcholine, du tocophérol et un solvant organique choisi parmi l'éthanol, le propylène glycol, la glycérine, le polyéthylène glycol, l'alcool benzylique, le diméthylacétamide et le diméthylsulfoxyde, dans laquelle :
1) le rapport de concentration du mélange entre ladite dioléylphosphatidylcholine et ledit tocophérol se situe dans la plage de 75/25 à 25/75,
2) la concentration dans le mélange de ladite dioléylphosphatidylcholine se situe dans la plage de 15 à 85 % (p/p),
3) la concentration dans le mélange dudit tocophérol se situe dans la plage de 15 à 85 % (p/p), et
4) la phase de la composition liquide non aqueuse devient un cristal liquide non lamellaire lors du contact avec l'eau, un tampon de phosphate, un fluide corporel, un fluide lacrymal ou un fluide vitré.

2. Composition liquide non aqueuse dissoute dans un médicament selon la revendication 1, dans laquelle :
1) le rapport de concentration du mélange entre ladite dioléylphosphatidylcholine et ledit tocophérol se situe dans la plage de 70/30 à 30/70,
2) la concentration dans le mélange de ladite dioléylphosphatidylcholine se situe dans la plage de 20 à 80 % (p/p), et
3) la concentration dans le mélange dudit tocophérol se situe dans la plage de 20 à 80 % (p/p).

3. Composition liquide non aqueuse selon la revendication 1 ou 2, dans laquelle ledit solvant organique est l'éthanol, l'alcool benzylique, le polyéthylène glycol ou le diméthylacétamide.

4. Solution injectable comprenant la composition liquide non aqueuse selon l'une quelconque des revendications 1 à 3.

5. Préparation ophtalmique comprenant la composition liquide non aqueuse selon l'une quelconque des revendications 1 à 3.
